Europäisches Patentamt

European Patent Office

Office européen des brevets

(11) Publication number: **0 259 739**
**A1**

## (12) EUROPEAN PATENT APPLICATION

(21) Application number: **87112654.6**

(22) Date of filing: **31.08.87**

(51) Int. Cl.⁴: **C12N 1/04**

(30) Priority: **10.09.86 US 905497**

(43) Date of publication of application:
**16.03.88 Bulletin 88/11**

(84) Designated Contracting States:
**DE FR IT**

(71) Applicant: **MILES LABORATORIES, INC.**
**1127 Myrtle Street**
**Elkhart Indiana 46514(US)**

(72) Inventor: **Barach, Jeffrey Truxton**
**1714 Canterbury Drive**
**Elkhart, IN 46514(US)**
Inventor: **Enders, George Leonhard**
**22797 Bainbridge Drive**
**Elkhart, IN 46514(US)**
Inventor: **Kamara, Bassie Jabron**
**234 Village Drive**
**Mishawaka, IN 46545(US)**

(74) Representative: **Dänner, Klaus, Dr. et al**
**c/o Bayer AG Konzernverwaltung RP**
**Patentabteilung**
**D-5090 Leverkusen 1 Bayerwerk(DE)**

(54) **Improved stability of freeze-dried cultures.**

(57) Disclosed is a novel method for maintaining the viability of a freeze-dried, lactic acid bacteria concentrate over a period of time. The method involves adding a combination of citric acid and glucose or a carbohydrate containing glucose linkages to a liquid suspension containing the cells and a cryoprotective agent and then freeze-drying the cells to provide a stabilized culture concentrate.

EP 0 259 739 A1

## IMPROVED STABILITY OF FREEZE-DRIED CULTURES

Background of the Invention

The present invention relates to the production of improved microbiological cultures and particularly to the production of dried, viable cultures of lactic acid producing bacteria (lactic bacteria) that will retain their viability at normal (ambient) temperatures for extended periods of time.

The food processing industry employs the incorporation of viable lactic bacteria into a variety of food products for flavor/texture development and preservation. Accordingly, there is a substantial demand for preserved lactic bacteria cultures that are simple to use, low in cost and easy to maintain. Preservation of these bacteria is commonly achieved by deep freezing or lyophilization. Deep freezing in liquid nitrogen is effective and relatively inexpensive. However, storage and transportation of the frozen cultures is complicated by the necessity of keeping them in the frozen state such as by the use of insulated containers containing dry ice. Lyophilized cultures can be packaged in foil wrappers and stored at normal or only slightly reduced temperatures but undergo a reduction in viability (cell count) during the freeze-drying process with further losses in cell count during storage.

In order to maintain high cell counts in lyophilized cultures, the addition of cryoprotectants and stabilizers is typically employed. For example, Porubcan et al in U.S. Patent No. 3,897,307 disclose the addition of an ascorbate in combination with either glutamate or aspartate to the fermented culture prior to the drying step for purposes of stabilization. In U.S. Patent No. 3,677,897 there is disclosed a method for stabilizing lactic bacteria by emulsifying the cells while still in liquid suspension with acetylated monoglyceride and blending the emulsified medium with food ingredients containing glucose, a modified starch and a modified cellulose and cooling the blend to encapsulate the lactics. Silliker et al, in U.S. Patent No. 3,075,887, describe the stabilization of dried viable bacterial cultures by adding an expandable proteinaceous material to an aqueous culture medium containing the bacteria and subjecting the resultant composition to a whipping operation to form a stable foam. The foamed culture is then extruded onto a drying surface and dried whereupon it is reduced to the desired particle size for use.

Kilara et al in Cultured Dairy Products Journal, Vol. II (1976) describe the use of cryoprotective agents such as dimethyl sulfoxide, glycerol, whey powder, Difco Casitone, Difco Peptonized Milk, Difco Malt Extract and non-fat dry milk for the cryostabilization of bacteria during lyophilization.

Valdez et al in Applied & Environmental Microbiology, 45:302-304 (1983) report that adonitol in the suspending medium markedly protected the viability of lactic bacteria tested whereas other polyhydric alcohols (dulcitol, mannitol, m-inositol and sorbitol) were found to provide little or no protection.

In J. Appl. Bact., 35:423-429 (1972) the use of (1) milk and its products, (2) serum, (3) glycerol, (4) peptone, (5) carbohydrates, (6) basal medium for vitamin $B_{12}$ assay, (7) dextran and sodium glutamate, (8) dimethylsulfoxide and (9) yeast extract are described as known materials for the protection of bacteria during freeze drying. The authors present evidence that malt extract is also useful for this purpose.

In Cryobiology, 20:260-566 (1983) Valdez et al discuss the use of protective additives in the suspending medium to reduce the death rate of micro-organisms in frozen storage. They describe the addition of glycerol, casein hydrolysates, yeast extract or egg white to ripened milk cultures of lactic streptococci. They also describe the use of dimethyl sulfoxide, acetamide and malic acid as effective protective agents for various types of cells against damage during freezing and freeze drying and report on a comparative study of various cryoprotectants using different species of lactic bacteria.

Summary of the Invention

The present invention is an improvement in the method of preserving lactic acid bacteria by subjecting the cells in liquid suspension to reduced temperature and pressure to thereby freeze dry them. The improvement comprises adding to the liquid suspension a cryoprotective amount of a material which will substantially reduce cell loss during the freezing and drying steps. In addition to the cryoprotective material there is added a potentiating amount of a combination of citric acid and glucose or a combination of citric acid and a polymer containing glucose linkages.

## Description of the Invention

The method of the present invention is useful to significantly stabilize, i.e., enhance the viability of, various species of lactic acid producing bacteria which are preserved by freeze-drying. Thus, while Lactobacillus acidophilus is a species whose sale in the freeze-dried state is common, other species of lactic bacteria such as L. bulgaricus, L. helveticus, L. casei, L . lactis, L. plantarum, L. delbrueckii, L. thermophilus, L. fermenti, Pediococcus cerevisiae, P. acidilactici, Streptococcus lactis, S. cremoris, S. diacetylactis, S. facium, S. thermophilus, Bifidobacterium thermophilium, B. bifidus and B . longum can be freeze-dried in accordance with the present invention to provide greater cell survival rates both during the freeze drying operation and upon storage for relatively long periods of time.

The combination of stabilizers disclosed herein potentiate the action of each other so that the combination provides a product showing good cell viability both during the rigors of the freeze-drying process and upon storage. In practice, the cells are propagated from a seed culture in a suitable nutrient growth medium and concentrated such as by centrifugation and/or filtration to provide a liquid culture concentrate to which is added the stabilizing materials. The initial concern with cell viability is their ability to withstand the freezing and drying procedures. The irreversible damage to the bacterial cells caused by the rigors undergone during the lyophilization procedure results in a large proportion of the cells being killed during freeze-drying. The initial loss of viability can be reduced by adding a cryoprotective amount of a material which will substantially reduce cell loss during the freezing and drying steps. Suitable materials include sucrose, peptones, casitone, a salt of glutamic acid, glycerol, dimethyl sulfoxide, non-fat dry milk or a milk product such as casein or whey, fructose and maltose. A particularly suitable cryoprotective system involves the use of a peptone derived from the enzymatic digestion of casein (sometimes referred to as hydrolyzed casein) sold under the trademark N-Z-Amine® by Humko Sheffield. The preferred material due to its solubility characteristics and low cost is the peptone N-Z-Amine YTT. The amount of this material needed for suitable cryoprotection will typically range from about 3.3 to 67 weight percent of the dry weight of the cells being stabilized. Also useful for cryoprotection in combination with the hydrolyzed casein is a carbohydrate. Suitable carbohydrates include dextrose, sucrose and a maltodextrin; lactose is preferred. From about 33 to 133 weight percent of the carbohydrate based on the dry weight of the cells being stabilized should be added to the liquid culture concentrate before freeze-drying. When a lactic bacteria concentrate containing these cryoprotectants is freeze-dried, good cell viability is observed initially, however, the stability of the freeze-dried culture with time is less than what may be desired. Accordingly, a material is added to the liquid culture concentrate to potentiate the ability of the hydrolyzed casein and carbohydrate to stabilize the freeze-dried culture concentrate. We have discovered that citric acid in combination with glucose or a polysaccharide containing glucose linkages is an effective potentiator for known cryoprotective agents which, when used in combination therewith will increase the storage stability of the freeze-dried culture concentrate. In the practice of the invention, citric acid is employed in an amount of from about 3.3 to 13.3 weight percent of the dry weight of the cells whereas glucose or a polysaccharide containing glucose linkages would be added to the liquid culture concentrate in an amount of from about 6.6 to 53 weight percent of the dry cells. A mixture of glucose and citric acid is suitable for use as a potentiator in the context of the present invention. Also suitable, in combination with citric acid, is a carbohydrate containing glucose linkages such as a disaccharide, e.g., sucrose or a polysaccharide, e.g., maltodextrins, starch or cellulose. In an embodiment where the same carbohydrate such as sucrose or a maltodextrin is used as both the cryoprotectant and potentiator, the amount added would have to be increased to account for this dual function.

The present invention is further illustrated by the following examples in which the bacteria were cultured in a suitable growth media and harvested by standard techniques such as filtration and/or centrifugation. Enumeration of viable cells was determined by serial dilution of the freeze-dried culture using sterile peptone water (0.1% w/v) and surface plating on ATP (All Purpose Tween, Difco) medium. Colony forming units per gram (CFU/gm) were counted after incubation at 37°C in a 10% carbon dioxide atmosphere. Only plates containing between 30 and 300 colonies were counted.

## Example I

Forty milliliters of freshly harvested liquid concentrate of Lactobacillus acidophilus (15 weight percent cells) at 4°C was adjusted to pH 6.5 with 50% NaOH. The concentrate was held at 4°C for 30 minutes to allow for equilibration and freeze-dried. The freeze-dried culture's stability was determined to be as follows:

3

## TABLE I

### STORAGE STABILITY RESULTS AT 22°C
### NO CRYOPROTECTANTS

| DAYS | CFU/gm | % RECOVERY |
|------|--------|------------|
| 0 | $3.1 \times 10^{10}$ | - |
| 7 | $1.3 \times 10^{10}$ | 42 |
| 14 | $2.6 \times 10^{9}$ | 8 |
| 21 | $2.4 \times 10^{8}$ | 0.8 |

Example II

A. Forty milliliters of a freshly harvested liquid concentrate of L acidophilus tempered to 4°C was mixed with 10 milliliters of solution #1. Solution #1 was prepared as follows:

7.5 gm of a peptone (NZ Amine® YTT - Sheffield Products, P.0. Box 630, Norwich, New York) and 40 gm of anhydrous lactose (Foremost) dissolved in 100 ml of distilled water to give 7.5% w/v and 40.0% w/v, respectively.

This gave a final concentration of 1.5% w/v NZ amine YT and 8% w/v lactose. The pH was adjusted to 6.5 with 50% NaOH.

B. Forty milliliters of a freshly harvested liquid concentrate of L. acidophilus at 4°C was mixed with 10 milliliters of solution #1 and held at 4°C for 30 minutes at which point 50 milliliters of solution #2 was added. Solution #2 was prepared as follows:

8.0 gm of a polysaccharide structurally between dextrin and maltose (Maltodextrin M100, Grain Processing Corporation, Muscatine, Iowa) and 1.3 grams citric acid - anhydrous fine powder (Miles Laboratories, Elkhart, Indiana) dissolved in 100 milliliters of distilled water to give a final concentration of 8% w/v and 1.3% w/v, respectively.

This gave a final concentration of 4.0% w/v Maltodextrin M100 and 0.65% w/v citric acid in the final composition. The pH was adjusted to 6.5 with 50% NaOH and the composition was held at 4°C for another 30 minutes to allow for equilibration.

C. Forty milliliters of a freshly harvested liquid concentrate of L. acidophilus at 4°C was mixed with 10 milliliters of solution #1 and held at 4°C for 30 minutes. Then 50 milliliters of solution #3 was added with mixing. Solution #3 was prepared as follows:

8 grams of D-glucose anhydrous (Mallinckrodt, Inc. Paris, Kentucky) and 1.3 grams citric acid anhydrous fine powder were dissolved in 100 milliliters of distilled water to give a final concentration of 8% w/v and 1.3% w/v, respectively.

Frozen pellets of each of the samples (A, B and C) were obtained by allowing the formulations to drip from a burette into liquid nitrogen. Freeze drying of the frozen pellets was carried out in a Virtis 10 SRC or an FTS Dura Dry freeze drying apparatus using procedures typically employed for freeze-drying cultures. The resulting freeze-dried pellets were crushed and stored at 4°C and 22°C in air using Naska polyethylene Whirl Pak bags. Neither nitrogen flushing nor vacuum headspace were employed.

Viability results after storage in air at 22°C for preparations A, B and C are set out in Table II.

4

## TABLE II

A.   NZ Amine + Lactose

| Days | CFU/gm | % RECOVERY |
|---|---|---|
| 0 | $1.3 \times 10^{11}$ | – |
| 7 | $2.0 \times 10^{11}$ | 100 |
| 17 | $7.9 \times 10^{10}$ | 60 |
| 24 | $3.4 \times 10^{10}$ | 26 |
| 31 | $2.7 \times 10^{10}$ | 20 |

B.   NZ Amine + Lactose + Maltodextrin M100
     + Citric Acid

| Days | CFU/gm | % RECOVERY |
|---|---|---|
| 0 | $1.2 \times 10^{11}$ | – |
| 7 | $1.2 \times 10^{11}$ | 100 |
| 17 | $1.1 \times 10^{11}$ | 92 |
| 24 | $7.9 \times 10^{10}$ | 66 |
| 31 | $7.6 \times 10^{10}$ | 63 |

C.   NZ Amine + Lactose + Glucose + Citric Acid

| Days | CFU/gm | % RECOVERY |
|---|---|---|
| 0 | $9.6 \times 10^{10}$ | – |
| 7 | $1.8 \times 10^{11}$ | 100 |
| 17 | $9.0 \times 10^{10}$ | 94 |
| 24 | $1.2 \times 10^{11}$ | 100 |
| 31 | $1.1 \times 10^{11}$ | 100 |

From the above data, it can be determined that formulations B and C provided improved storage stability as compared to formulation A. After 31 days, significantly greater loss of viability was observed for formulation A than for formulations B or C, i.e., 20% recovery -vs-63 and 100% recovery, respectively.

It should be noted that in runs B and C the NZ-amine/lactose was added first and the citric acid/carbohydrate potentiator added after equilibration had been allowed to take place. Adding all four ingredients to the liquid culture concentrate at one time produced less favorable results. Likewise, the addition of NZ-amine, lactose and citric acid followed, after equilibration, by the addition of a carbohydrate potentiator is not entirely suitable. However, it has been found that the addition of NZ-amine, lactose and the carbohydrate followed by citric acid after equilibration provides good results in terms of maintaining cell viability during freeze-drying.

Example III

A. Forty milliliters of a freshly harvested liquid concentrate of L. acidolphilus at 4°C was mixed with 10 milliliters of solution #1 and held at 4°C for 30 minutes whereupon 50 milliliters of solution #4 was added. Solution #4 was prepared as follows:

8 grams of D-glucose anhydrous (Mallinckrodt, Inc.) was dissolved in 100 milliliters of distilled water to give a final concentration of 8.0% w/v.

This provided 4.0% w/v of D-glucose in the final composition. The pH was adjusted to 6.5 with 50% NaOH and held at 4°C for 30 minutes to allow for equilibration.

B. Forty milliliters of a freshly harvested liquid concentrate of L. acidophilus at 4°C was mixed with 10 milliliters of solution #1 and held at 4°C for 30 minutes whereupon 50 milliliters of solution #5 was added. Solution #5 was prepared as follows:

8 grams of Maltodextrin 100 (Grain Processing Corp., Muscatine, Iowa) was dissolved in 100 milliliters of distilled water to give a final concentration of 8.0% w/v.

This provided 4.0% w/v of Maltodextrin M100 in the final composition. The pH was adjusted to 6.5 with 50% NaOH and the composition was held at 4°C for 30 minutes to allow for equilibration.

C. Forty milliliters of a freshly harvested liquid concentrate of L. acidolphilus at 4°C was mixed with 10 milliliters of solution #1 and held at 4°C for 30 minutes whereupon 50 milliliters of solution #6 was added. Solution #6 was prepared as follows:

2.6 grams citric acid anhydrous fine powder (Miles Laboratories, Inc.) was dissolved in 100 milliliters of distilled water to give a final concentration of 2.6% w/v.

This gave a 1.3% w/v of citric acid in the final composition. The pH was adjusted to 6.5 with 50% NaOH. The composition was held at 4°C for 30 minutes to allow for equilibration.

The viability of the cells in these preparations was determined as before. These data are set out in Table III (A, B and C).

## TABLE III

### A.    NZ Amine + Lactose + Glucose

| Days | CFU/gm | % RECOVERY |
|---|---|---|
| 0 | $1.2 \times 10^{11}$ | – |
| 7 | $9.0 \times 10^{10}$ | 75 |
| 14 | $6.3 \times 10^{10}$ | 53 |
| 21 | $6.3 \times 10^{10}$ | 53 |
| 31 | $4.3 \times 10^{10}$ | 36 |

### B. NZ Amine + Lactose + M100

| Days | CFU/gm | % RECOVERY |
|------|--------|------------|
| 0 | $1.2 \times 10^{11}$ | - |
| 7 | $6.9 \times 10^{10}$ | 58 |
| 14 | $5.5 \times 10^{10}$ | 46 |
| 21 | $5.5 \times 10^{10}$ | 46 |
| 31 | $4.6 \times 10^{10}$ | 38 |

### C. NZ Amine + Lactose + Citric Acid

| Days | CFU/gm | % RECOVERY |
|------|--------|------------|
| 0 | $1.2 \times 10^{11}$ | - |
| 7 | $6.0 \times 10^{10}$ | 50 |
| 14 | $6.8 \times 10^{10}$ | 57 |
| 31 | $3.6 \times 10^{10}$ | 30 |

From the data presented in Tables II and III it can be determined that neither citric acid, glucose nor a carbohydrate containing glucose linkages provide significant stabilization by themselves. However, the data clearly demonstrate that the combination of citric acid and glucose or a carbohydrate containing glucose linkages acts as a potentiator for the cryoprotective material to stabilize the freeze-dried cultures over time.

**Claims**

1. A method for preserving lactic acid bacteria by subjecting a liquid medium containing viable cells of the bacteria and a cryoprotective amount of a material which will substantially reduce cell loss during freezing and drying at reduced pressure and temperature to thereby freeze dry the cells, characterized in adding to the liquid medium before such treatment a potentiating amount of a combination of citric acid and glucose or a carbohydrate containing glucose linkages.

2. The method of Claim 1 wherein the cryoprotective material is sucrose, a peptone, casitone, a salt of glutamic acid, glycerol, dimethyl sulfoxide, non-fat dry milk, casein, whey, fructose or maltose.

3. The method of Claim 1 wherein the cryoprotective material is a combination material 3.3 to 67 weight percent of a peptone derived from the enzymatic digestion of casein and 33 to 133 weight percent of a carbohydrate selected from the group of dextrose, sucrose, a maltodextrin and lactose where all percentages are based on the dry weight of the cells.

4. The method of Claim 3 wherein the carbohydrate is lactose.

5. The method of Claim 1 wherein the amount of citric acid added is from about 3.3 to 13.3 weight percent and the amount of glucose or carbohydrate containing glucose linkages added is from about 6.6 to 53 weight percent where all percentages are based on the dry weight of the cells.

6. The method of claim 5 wherein the carbohydrate is a maltodextrin.

7. A method of preserving a culture concentrate of lactic acid bacteria to thereby maintain cell viability over a period of time which method comprises:

a) providing an aqueous suspension of the cells to be preserved,

b) adding:

i. a combination of 3.3 to 67 weight percent of a peptone derived from the enzymatic digestion of casein and 33 to 133 weight percent of lactose, and

ii. a combination of 3.3 to 13.3 weight percent citric acid and 6.6 to 53 weight percent of glucose or a carbohydrate having glucose linkages as potentiator

to the aqueous suspension where all percentages are based on the dry weight of the cells, and

c) subjecting the aqueous suspension to reduced temperature and pressure to thereby provide a stabilized, freeze-dried culture concentrate of the lactic acid bacteria.

8. The method of Claim 7 wherein the potentiator is glucose.

9. The method of Claims 1-8 wherein the lactic bacteria is selected from the group of Lactobacillus acidophilus, L. bulgaricus, L. helveticus, L. casei L. lactis, L. plantarum, L. delbrueckii , L. thermophilus, L. fermenti, Pediococcus cerevisiae, P. acidilactici, Streptococcus lactis, S. cremoris, S . diacetylactis, S. faecium, S. thermophilus, Bifidobacterium thermophilium, B. bifidus and B. longum.

10. The method of claims 1-8 wherein the lactic bacteria is of the species L. acidophilus.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF DAIRY SCIENCE, vol. 57, no. 2, February 1974, pages 165-173, Champaigne, US; C.A. SPECKMAN et al.: "Lyophilized lactic acid starter culture concentrates: preparation and use in inoculation of vat milk for cheddar and cottage cheese" * Page 165, right-hand column, paragraph 2 - page 166, left-hand column, paragraph 5 * | 1,2,5, 9 | C 12 N 1/04 |
| A | US-A-4 199 022 (S.M. SENKAN et al.) * Claims 1,5 * | 1 | |
| A | US-A-4 226 940 (A.B. STORRS) -* Claims 1,8,9 *. | 1,2 | TECHNICAL FIELDS SEARCHED (Int. Cl.4) |
|  |  |  | C 12 N |

The present search report has been drawn up for all claims

| Place of search | Date of completion of the search | Examiner |
|---|---|---|
| THE HAGUE | 16-10-1987 | WIESER, M.R.J. |